# EUROPEAN PATENT SPECIFICATION

(11) **EP 2 648 642 B1**
(45) Date of publication and mention of the grant of the patent: **10.09.2014**
(21) Application number: 11820817.2
(22) Date of filing: 05.12.2011
(51) Int. Cl.: A61B 19/00, A61B 17/326, A61M 29/00

(54) **MEDICAL DEVICE FOR THE PREVENTION AND/OR CORRECTION OF PHIMOSIS THROUGH THE STATIC PROGRESSIVE DILATATION OF SCAR RING**
MEDIZINISCHE VORRICHTUNG ZUR PRÄVENTION UND/ODER KORREKTUR VON PHIMOSE DURCH STATISCHE PROGRESSIVE DILATATION EINES NARBENRINGS
DISPOSITIF MÉDICAL DE PRÉVENTION ET/OU DE CORRECTION DU PHIMOSIS PAR DILATATION PROGRESSIVE STATIQUE DU PRÉPUCE

(30) Priority: 06.12.2010 IT RM20100632
(43) Date of publication of application: 16.10.2013
(73) Proprietor: Boschetto, Carlo, 00119 Roma (IT)
(72) Inventor: Boschetto, Carlo, 00119 Roma (IT)
(74) Representative: Marcio', Paola
(86) International application number: PCT/IT2011/000407
(87) International publication number: WO 2012/077151

(56) References cited:
- CN-A- 101 703 429
- CN-Y- 201 120 045
- DE-U1-202010 009 101
- SU-A1- 888 981
- US-A- 2 085 368
- US-A1- 2007 043 388

## Description

The present invention relates to the field of medical devices for the treatment of diseases concerning the male urogenital apparatus.

More in detail, the present invention concerns a medical device for the prevention and/or correction of phimosis through the static progressive dilatation of scar ring.

As it is known, the normal regenerative ability of the prepuce tissue, when affected by phimosis, is blocked by the cicatricial retraction produced by infections, technically called balanoposthitis, caused by viruses, bacteria or fungi.

As it is furthermore known, such cicatricial formation remains even after healing of the generating inflammation, preventing the prepuce from uncovering the glans and causing hygienic and functional problems, including hindering sexual activity due to pain and, in case of extreme phimosis, difficulty in urinating and other serious consequences.

The medical devices currently available for the treatment of phimosis generally have drawbacks that greatly limit their therapeutical effectiveness.

US 2 085 368 A discloses a spring device to be inserted between the glans and the prepuce, which has the defect of not being adjustable and/or carrying out a too violent action and that impair the local blood flow and may lead to necrosis.
Other known devices are based on couples of rods to be inserted between the glans and the prepuce, and continuing out of the same, where they are connected to each other by means of adjustable spacers, and are therefore cumbersome, bothersome, hinder urination and prone to spontaneous expulsion.

For such reasons, the elective cure for phimosis of medium or severe level is, very frequently, a circumcision operation.

US 2007/043388A1 discloses a set of small dilator tubes with progressively larger diameters but with same hight and shape, unsuitable to be adapted to the shape of the male genital organ.
In order to overcome the clinical problems concerning the therapeutical treatment of phimosis, as well as the limits that characterize the medical devices available at present for the treatment of said disease, a medical device has been developed, whose use according to a clinical protocol feasible therewith at first can produce a rarefaction of scar cells with consequent a weakening of the phimotic ring, and subsequently a growth of elastic cells that are interposed between the phimotic cells hindering regrouping, thus obtaining the restoration of elasticity and of the diameter of the prepuce to the levels present before the event that generated the onset of the phimosis.

As a result, circumcision is no longer necessary.

Said medical protocol has been developed starting from the assumption that the prepuce tissue has the natural ability to regenerate that are effective in the case of minor diseases and/or diseases that are treated early but, in the presence of balanoposthites which are more aggressive or simply overlooked, they are evidently incapable to naturally restore the tissue functionality of the prepuce previous to the cicatricial retraction.

Following said assumption, it has been surprisingly discovered that a small dilation artificially produced on the prepuce lumen constricted by phimosis, and kept in place for a sufficient amount of time, restores both a larger prepuce lumen and partial elasticity starting from the new diameter of the lumen obtained by constriction, and it also generates an initial partial remission of the phimosis.

Besides, reapplying the application numerous times with suitable constrictions that have an increasingly larger diameter starting at each new dimension reached by the prepuce lumen, it has been surprisingly noticed the restoration of the lumen existing before the onset of the inflammatory events, or a lumen that is physiologically necessary, as well as attaining the previous natural elasticity of the prepuce tissues and complete remission of the phimosis.

Finally it has been noticed the mobilization of scarring, and a longterm disappearance of the phimosis.

The above described medical protocol is also effective as a means of prevention, since as soon as inflammation of the prepuce occurs, together with the application of common active substances prescribed by a doctor for the specific infectious agent, a mere constriction with a larger diameter is placed, not for dilatation purpose but for conservative purpose, to be kept in place until the infection is healed. In such a way the possible evolution of the inflammation into phimosis is made impossible, even if it has been clinically solved.

Finally, in individuals with a tendency of relapsing phimosis even in the absence of inflammatory events, the use of the device through periodical execution of said medical protocol allows to ensure permanent preservation of the lumen and of the elasticity of the prepuce.

According to what described above, it is therefore aim of the present invention to realize a medical device made of a set of tube-shaped objects, thereinafter called "small tubes", arranged for being worn in order on the glans and around it, so as to allow the correct implementation of the above-described protocol for the prevention and/or correction of phimosis through the static progressive dilatation of the prepuce scar ring.

The aim is reached by means of a medical device for the prevention and/or correction of phimosis through the static progressive dilatation of the prepuce scar ring, as claimed in claim 1.

The dependent claims define further features of the present invention.

The invention has the following numerous advantages:
- it allows to obtain the remission of phimosis and its inelastic cicatricial retraction, without surgical circumcision;
- it allows to prevent the development of phimosis as a result of inflammatory diseases of the prepuce tissue;
- it allows to maintain the lumen and the optimal elasticity of the prepuce in individuals with a tendency to relapsing phimosis;
- it allows to correct congenital constricted prepuce that prevents the glans from being correctly uncovered.

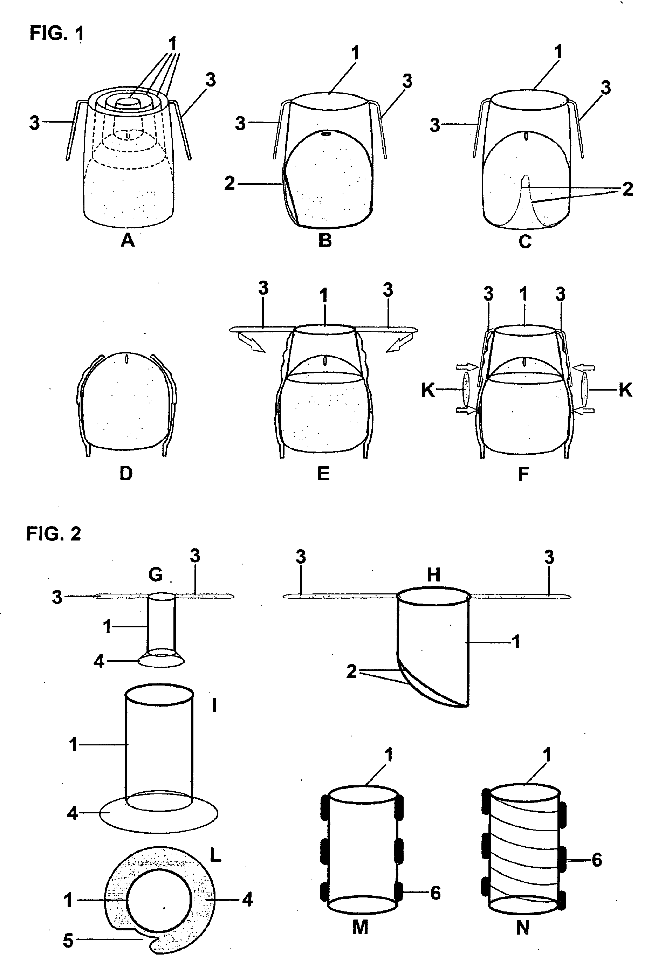

Further characteristics and advantages of the present invention shall appear more clearly from the following description of a preferred embodiment, made by way of an indicative and non-limiting example, with reference to the figures, wherein
figure 1 shows:
   - in A, an axonometric and partly transparent view of a set of small tubes positioned on the glans in order to point out the different dimentional relation with the surface thereof;
   - in B e C, a respectively lateral and front axonometric view of a larger small tube inserted up to the coronal sulcus of the glans;
   - in D, a partial vertical section of the position of the phimotic prepuce on the glans;
   - in E, an axonometric view and partial vertical section of an intermediate small tube on the walls of which the prepuce has been pulled up and whose fixing flaps are unfolded;
   - in F, an axonometric view of an intermediate small tube whose fixing flaps are folded beside the prepuce and fixed thereto by means of patches;
figure 2 shows:
   - in G, an axonometric view of a small-sized small tube comprising a lower conical ring arranged for preventing the scar ring of the prepuce to move from its seat;
   - in H, an axonometric view of a larger small tube heavily modified for being anatomically adapted to the area in contact with the glans coronal sulcusand to the area where the frenulum is;
   - in I and L, respectively an axonometric view and a top plan view of a small tube comprising a lower ring partially interrupted in order to facilitate the insertion thereof into the prepuce lumen by twisting, therefore with less need of dilatation thereof during placement;
   - in M an axonometric view of a small tube provided with grooves and/or ridges arranged for keeping the prepoce in place;
   - in N an axonometric view of a small tube provided with grooves and/or ridges arranged for facilitating the raise of the prepuce along the smal tube itself, by suitable screwing.

With reference to the details of figures 1 and 2, the medical device according to the present invention is composed by a set of small tubes 1, that can be worn progressively on the glans and around it, provided with progressively growing diameter and hight, and anatomically shaped, with shapes 2 that are progressively more incised, in order to be adapted without difficulties to the morphology of the glans, of the frenulum and of the glans coronal sulcus.

Said small tubes 1 further comprise:
- flexible top flaps 3, arranged for being folded out of the prepuce and fixed thereto, by means of patches K or similar means, in order to keep the small tubes 1 in the correct position of placement on the glans;
- a lower conical ring 4, arranged for keeping the scar ring of the prepuce around the small tubes 1, preventing the same from slipping downwards, comprising a partial discontinuity 5 of its own structure, arranged for facilitating the insertion of the small tubes, by twisting, into the prepuce lumen with a smaller necessity of dilatation thereof;
- grooves and/or ridges 6, arranged on the outer surface of the small tubes 1, arranged for allowing the rise of the prepuce along the same, by screwing, or keeping it in position.

According to the present invention, a first small tube 1, whose dimentions are arranged for allowing insertion thereof into the prepuce lumen without eccessive forcing, is placed on the glans, in such a way as to allow the rise of the scar ring of the prepuce over the conical, ring 4, placed in correspondance with the lower edge of said small tube 1.

The placement of the small tube 1 on the glans determines an advantageous mecanical dilatation of the scar ring of the prepuce rising along the tubular structure thereof, and is suitably prolonged over time until survey of a first stage of regression of the phimosis, functional to the placement of a subsequent small tube of a larger size.

Repetition of the above mentioned procedure, carried out by means of small tubes 1 with progressively increasing sizes, allows to achieve enervation of the scar ring functional to the achievement of the lumen and of the elasticity of the prepuce, and therefore of its complete functionality, thus determining total remission of the phimosis. in progress, without circumcision surgery.

During repetition of the above described procedure, the different dimentions and hights of the small tubes 1, as well as the different anatomical shapes thereof, having shapes 2 progressively more incised, allow easy adjustment thereof first on top of the glans, then around the same, leaving the frenulum area free, and finally on the glans coronal sulcus.

The steady and durable placement of the small tubes 1 on the glans is made possible by suitable flexible top flaps 3, arranged for being folded out of the prepuce rising along the tubular structure of said small tubes 1, and fixed thereto by means of patches K, or similar means.

A partial discontinuity 5 of the relevant conical ring 4 allows to facilitate the positioning of the small tubes 1 on the glans by suitable twisting, and therefore with a smaller necessity of dilatation of the preputial lumen, while suitable grooves and/or ridges 6, arranged on the outer profile of said small tubes 1, allow the rising of the prepuce along the tubular structure thereof, by suitable scewing, or merely keep it in position.

Besides allowing therapeutic treatment of phimosis, said small tubes 1 can represent advantageous prevention against the onset of such disease.

In case of preputial inflammatory events, potentially capable of produce phimotic results and inelasticity due to inelastic cicatricial retractions, such evolution can be indeed prevented by early use only of the small tubes 1 of larger size, which will help the preputial tissue not to deteriorate, shrink and cicatrize, during the attack of the infective agents, while the drugs commonly prescribed by the doctor will act to heal the infection.

Besides, the individuals subject to relapsing phimosis even without inflammatory emergence will be able to maintain a normal lumen and a normal elasticity of the prepuce wearing the small tubes 1 at regular intervals.

Said small tubes 1 are preferably made of plastic anallergic materials of medium elasticity, and by means of common extrusion or moulding, are washable by common disinfectant cleansers, and can be kept in the established placement seats during urination.

## Claims

1. Medical device for the prevention and/or correction of phimosis through the static progressive dilatation of the prepuce scar ring, comprising:
- a set of dilator small tubes (1) having progressively larger diameter;
wherein
said small tubes (1) have different shape and height, so as to be anatomically configured to be adapted at first to the end of the glans, with the smaller sizes, then around said glans with the intermediate sizes, with shapes (2) that are progressively more incised to leave the frenulum area free, and finally, with the larger sizes, to be positioned on the glans coronal sulcus according to its morphology
and wherein at least one of the small tubes (1) of said set comprise flexible top flaps (3), that can be folded downwards around the prepuce and that can be fastened thereto by means of patches (K) or similar, for the double purpose of preventing the prepuce from overhanging the small tubes (1) shrinking thereon and keeping said small tubes (1) in place during use.

2. Device according to claim 1, **characterized in that** at least one of the small tubes (1) of said set comprise a lower conical ring (4) having the purpose of keeping the scar ring around the small tubes (1), preventing the same from slipping downwards, thus avoiding the dilating action.

3. Device according to claim 2, **characterized in that** the lower ring (4) comprise a partial discontinuity (5) of its own structure, arranged for facilitating the insertion of the small tubes (1), by twisting, into the prepuce lumen with a smaller necessity of dilatation thereof during the placement.

4. Device according to claim 1, **characterized in that** at least one of the small tubes (1) of said set comprise grooves and/or ridges (6) arranged for allowing the rise of the inner surface of the prepuce along the same, by screwing, or keeping it in position.

## Patentansprüche

1. Medizinische Vorrichtung zur Prävention und/oder Korrektur der Phimose durch statische progressive Dilatation des Narbenrings an der Vorhaut, umfassend:
- ein Set von kleinen Dilatator-Röhrchen (1) mit progressiv steigendem Durchmesser; wobei die besagten kleinen Röhrchen (1) eine verschiedene Form und Höhe aufweisen und anatomisch so gestaltet sind, dass sie mit den kleineren Größen zuerst an das Ende der Eichel angepasst werden können, dann rund um die besagte Eichel mit den mittleren Größen mit Formen (2), die progressiv eingeschnittener sind, um den Bereich des Frenulums frei zu lassen, und schließlich, dass sie gemäß ihrer Morphologie mit den größten Größen auf die Sulcus coronalis der Eichel positioniert werden können und wobei mindestens eines der kleinen Röhrchen (1) dieses Sets flexible obere Klappen (3) umfassen, die nach unten rund um die Vorhaut gefaltet werden können und die daran durch Patchs (K) oder Ähnliches daran befestigt werden können, zum Doppelzweck, damit die Vorhaut vom Überhängen über die kleinen Röhrchen (1) gehindert wird, sich daran zusammenzieht und die besagten keinen Röhrchen (1) während des Gebrauchs an ihrem Platz hält.

2. Vorrichtung gemäß Anspruch 1, **gekennzeichnet dadurch, dass** mindestens eines der kleinen Röhrchen (1) des besagten Sets einen unteren konischen Ring (4) umfasst, der den Zweck verfolgt, den Narbenring rund um die kleinen Röhrchen (1) zu halten, indem er denselben davon abhält, nach unten zu gleiten und auf diese Weise die erweiternde Wirkung verhindert.

3. Vorrichtung gemäß Anspruch 2, **gekennzeichnet dadurch, dass** der untere Ring (4) eine Teildiskontinuität (5) seiner eigenen Struktur beinhaltet, angeordnet zur Erleichterung der Einfügung der kleinen Röhrchen (1) durch Eindrehen in das Vorhautlumen, mit einer kleineren Notwendigkeit der Dilatation dieses während der Positionierung.

4. Vorrichtung gemäß Anspruch 1, **gekennzeichnet dadurch, dass** mindestens eines der kleinen Röhrchen (1) des besagten Sets Rillen und/oder Grate (6) beinhaltet, die so angeordnet sind, dass sie den Anstieg der Innenfläche der Vorhaut längs derselben durch Drehen erlauben oder sie in der Lage halten.

## Revendications

1. Dispositif médical de prévention et/ou de correction du phimosis par dilatation progressive et statique de l'anneau cicatriciel du prépuce, comprenant :
- un set de petits tubes de dilatateur (1) ayant progressivement un diamètre plus grand; où lesdits petits tubes (1) possèdent une forme et une hauteur différentes, de manière à être configurés anatomiquement pour être adaptés premièrement sur l'extrémité du gland, avec les tailles plus petites, puis autour dudit gland avec les tailles intermédiaires, avec des formes (2) qui sont progressivement plus incisées pour laisser libre la zone du frein, et enfin, avec les tailles plus grandes, pour être positionnés sur la scissure coronale du gland en fonction de sa morphologie, et où au moins un des petits tubes (1) dudit set comprend des rabats supérieurs flexibles (3), qui peuvent être repliés vers le bas autour du prépuce et qui peuvent y être fixés au moyen de pièces (K) ou similaires, pour le double but d'empêcher le prépuce de surplomber les petits tubes (1) qui se rétrécissent et de maintenir lesdits petits tubes (1) en place durant l'utilisation.

2. Dispositif selon la revendication 1, **caractérisé par le fait qu'**au moins un des petits tubes (1) dudit set comprend une bague conique inférieure (4) ayant pour but de maintenir l'anneau cicatriciel du prépuce autour des petits tubes (1), empêchant celui-ci de glisser vers le bas, évitant ainsi l'action de dilatation.

3. Dispositif selon la revendication 2, **caractérisé par le fait que** la bague inférieure (4) comprend une discontinuité partielle (5) de sa propre structure, prévue pour faciliter l'insertion des petits tubes (1), par torsion, dans l'espace du prépuce avec une nécessité de dilatation inférieure de celui-ci durant le placement.

4. Dispositif selon la revendication 1, **caractérisé par le fait qu'**au moins un des petits tubes (1) dudit jeu comprend des rainures et/ou des arêtes (6) prévues pour permettre le soulèvement de la surface interne du prépuce le long de celles-ci, par vissage, ou maintien en position.
